# EUROPÄISCHE PATENTSCHRIFT

(11) **EP 1 413 322 B1**
(45) Veröffentlichungstag und Bekanntmachung des Hinweises auf die Patenterteilung: **05.07.2006**
(21) Anmeldenummer: 03090349.6
(22) Anmeldetag: 16.10.2003
(51) Int. Cl.: A61L 27/12

(54) **Phosphathaltiger Knochenersatzwerkstoff mit kristallinen und röntgenamorphen Phasen**
Phosphate containing bone substitute product with crystalline and amorphous phases
Matériau pour prothèse osseuse à base de phosphate comprenant des phases cristallines et amorphes

(30) Priorität: 21.10.2002 DE 10249626
(43) Veröffentlichungstag der Anmeldung: 28.04.2004
(73) Patentinhaber: BAM Bundesanstalt für Materialforschung und -prüfung, 12205 Berlin (DE)
(72) Erfinder: Berger, Georg, 16341 Zepernick (DE); Spitzer, Andrea, 10827 Berlin (DE); Jäger, Christian Prof., 07749 Jena (DE); Pauli, Jutta, 12555 Berlin (DE); Gildenhaar, Renate, 13086 Berlin (DE)
(74) Vertreter: Walter, Wolf-Jürgen

(56) Entgegenhaltungen:
- WO-A-91/07357
- DE-C- 19 744 809
- US-A- 3 922 155

## Beschreibung

Die Erfindung betrifft ein orthophosphathaltiges Material mit hoher Löslichkeit, das sowohl als bioaktiver Knochenersatzwerkstoff, so z.B. spongiosa-ähnliches Material, als Beschichtung von metallischen Prothesenstielen durch thermisches Spritzen, durch rf-Sputtering, als auch als Substratmaterial in der Biotechnologie, speziell dem Tissue Engineering, z.B. auch als keramische Folie, als kompakter oder poröser, d.h. spongiosa-ähnlicher "scaffold"-artiger, Formkörper Anwendung finden kann. Die Erfindung betrifft auch ein Herstellungsverfahren.
Anorganische Materialien mit hoher Resorbierbarkeit sind an sich bekannt. Auch Werkstoffe, die ihren speziellen Einsatz als bioaktive Knochenersatzwerkstoffe finden und eine schnelle Löslichkeit besitzen, sind in der Literatur beschrieben. Beispielsweise wurde ständig über den erfolgreichen klinischen Einsatz von Keramiken mit den Hauptkristallphasen alpha- oder beta-Tricalciumphosphat (TCP) berichtet. Zudem gab es auch vergleichende Untersuchungen dieser beiden TCP-Phasen im Tierversuch. Aus EP 237043 ist bekannt, dass an der Oberfläche von aus alpha-TCP hergestellten Granulaten Dicalciumphosphat enthalten ist, was besonders in der Anfangsphase nach der Implantation eine höhere Löslichkeit aufwies als das reine Kernmaterial aus alpha-TCP.

Übertroffen wurde deren chemische Löslichkeit durch ebenfalls bioaktive Werkstoffe auf der Basis von Calciumphosphaten, die zusätzlich Oxide des Kaliums, Natriums, Magnesiums und/oder Siliciums enthalten (EP 541546 B1) und deren glasig-kristallines Material sich auf die folgende Hauptkristallphasen gründet: Phase X, Rhenanit, Phase nach Ando (Phase A) bzw. von diesen zuvor genannten Phasen abgeleitete Mischkristalle zwischen diesen Kristallphasen enthält.

Der Erfindung liegt die Aufgabe zugrunde, ein neues orthophosphathaltiges Material bereitzustellen, das weitere Phosphate enthält und einen überwiegend direkten, bindegewebsfreien Knochenverbund bzw. die Ex-vivo-Kultivierung von Knochenzellen ermöglicht und sich im Kontakt mit Knochengewebe auflöst und dabei verbessert einstellbare Löslichkeiten und bei Kompositen an bestimmte Metalle angepasste Ausdehnungskoeffizienten aufweist. Eine weitere Aufgabe der Erfindung besteht in der Bereitstellung eines Herstellungsverfahrens für dieses Material.

Erfindungsgemäß umfasst der Knochenersatzwerkstoff a) nach ³¹P-NMR-Messungen Q₀-Gruppen von Orthophosphat und Q₁-Gruppen von Diphosphat, wobei die Orthophosphate respektive Q₀-Gruppen, bezogen auf den Gesamtphosphorgehalt des fertigen Werkstoffes, 65 bis 99,9 Gew-% betragen, und die Diphosphate respektive Q₁₋Gruppen, bezogen auf den Gesamtphosphorgehalt des fertigen Werkstoffes, 0,1 bis 35 Gew-% betragen, und
b) nach röntgendiffraktometrischen Messungen, bezogen auf das Gesamtgewicht des fertigen Werkstoffes, 35 bis 99,9 Gew-% einer Hauptkristallphase aus Ca₁₀Na(PO₄)₇, Ca₁₀K(PO₄)₇, Gemische davon oder Mischkristalle im Umfang von Ca₁₀KₓNa₁₋ₓ(PO₄)₇ mit x=0 bis 1, und als Nebenkristallphase, bezogen auf das Gesamtgewicht des fertigen Werkstoffes, 0,1 bis 25 Gew-% eines Stoffes, ausgewählt aus der Gruppe, bestehend aus Na₂CaP₂O₇, K₂CaP₂O₇, Ca₂P₂O₇ und Gemische davon, und
c) röntgenamorphe Phasen, die neben der Hauptkristallphase insgesamt 0,1 bis 65 Gew-% betragen, bezogen auf das Gesamtgewicht des fertigen Werkstoffes, erhältlich durch Vermischen von Rohstoffen, enthaltend (in Gew-%) 30-50 CaO, 1-12 Na₂O, 0,5-15 K₂O, 1,5-5 MgO, 0-5 SiO₂ und Behandlung mit H₃PO₄ entsprechend einem Anteil von 35-55 P₂O₅, wobei SiO₂ oder MgO oder ein Gemisch davon wenigstens 1 Gew-% beträgt, das Gemenge homogenisert und getrocknet wird und einer stufenweisen Temperaturbehandlung von jeweils 1-2h bei 350-450 °C, 750-850 °C und 950-1050 °C unterworfen wird, und das Gemisch bei 1550 bis 1650 °C geschmolzen, bei der Schmelztemperatur 10 bis 60 Minuten gehalten und schließlich spontan oder mit geregelter Temperatur abgekühlt und gegebenenfalls gemahlen und zu Formkörpern gesintert wird.

x ist vorzugsweise 0,1 - 1, insbesondere 0,2 -1.

Bevorzugt sind in der Nebenkristallphase oder in der amorphen Phase zusätzlich ein oder mehrere Stoffe der Gruppe β-Ca₃(PO₄)₂, Ca₂K₁₋ₓNa₁₊ₓ(PO₄)₂ (x=0,1 - 1),

CaNaPO₄, NaPO₃, KPO₃ und Mischkristalle davon enthalten, wobei die Kettenphosphate NaPO₃ und KPO₃ nach ³¹P-NMR-Messungen als Q₂-Gruppen nachzuweisen sind und die Orthophosphate nach ³¹P-NMR-Messungen als Q₀₋Gruppen nachzuweisen sind.

Weiterhin kann in der Nebenphase entsprechend dem SiO₂-Anteil eine Silicatphase in einem Anteil bis 6 Gew-% enthalten sein.

In der o.g. Hauptkristallphase sowie in den Bestandteilen der Nebenkristallphase kann in Mischkristallen das Element Ca durch Mg bis zu einem Anteil von 10 Gew-% ersetzt sein, bezogen auf das Gewicht des fertigen Werkstoffs.

Der Anteil der Orthophosphatphase als Q₀-Gruppen kann bevorzugt im Bereich von 40 bis 95 Gew-% liegen, insbesondere 50 bis 90 Gew-%.

Der Anteil der Diphosphatphase als Q₁-Gruppen kann bevorzugt im Bereich von 1 bis 22 Gew-% liegen, insbesondere im Bereich von 5 bis 8 Gew-%.

Die Zusammensetzung des erfindungsgemäßen Materials auf Basis von CaO, P₂O₅, Na₂O, K₂O, und ggf. auch MgO sowie SiO₂, das als röntgenamorph - kristallin anzusehen ist, liegt im Bereich von (in Gew-%):

| | |
|---|---|
| 35 bis 55 | P₂O₅ ; 30 bis 50 CaO; |
| 1 bis 12 | Na₂O; 0,5 bis 15 K₂O; |
| 0 bis 5 | MgO, vorzugsweise 0,1-5 MgO; |
| 0 bis 5 | SiO₂; wobei MgO oder SiO₂ oder ein Gemisch davon wenigstens 1 Gew-% beträgt. |

Die Zusammensetzung enthält als Hauptkristallphasen die o.g. Phasen und als kristalline Nebenbestandteile einen oder mehrere Bestandteile der Gruppe Na₂CaP₂O₇, K₂CaP₂O₇, Ca₂P₂O₇, β-Ca₃(PO₄)₂, Ca₂K₁₋ₓNa₁₊ₓ(PO₄)₂, CaNaPO₄, NaPO₃, und KPO₃, sowie eine röntgenamorphe Phase, wobei x=0,1 - 0,9 ist.

Ein bevorzugtes Material enthält folgende Bestandteile:
in Gew-%: 43 bis 55 P₂O₅ , 32 bis 48 CaO , 1,5 bis 11 Na₂O, 1,5 bis 12 K₂O, 0,5 bis 2 MgO, 0,0 bis 2 SiO₂ . Eine spezielle bevorzugte Ausführungsform enthält 44 bis 54 P₂O₅, 34 bis 48 CaO, 1,5 bis 10,5 Na₂O, 1 bis 11 K₂O, 1,5 bis 3 MgO , 0,1 bis 4 SiO₂.

Der hier verwendete Begriff "röntgenamorphes" Material ist im allgemeinen nicht eindeutig definierbar. Unter röntgenamorph wird hier ein Material verstanden, dessen Struktur mit der üblichen XRD (Röntgendiffraktometrie) nicht mehr erfasst werden kann und somit als röntgenamorph bezeichnet werden kann. Dabei kann es sich um sehr kleine geordnete Bereiche (mikrokristallin) als auch statistisch ungeordnete Bereiche handeln. Im Gegensatz zu XRD kann durch die ³¹P-NMR-Ergebnisse die Existenz jeder kristallinen Phase erfasst werden. Deshalb kann es bei der Mengenabschätzung zwischen NMR- und XRD-Ergebnissen zu gravierenden Unterschieden kommen. Besonders die Diphosphat- und Kettenphosphatanteile scheinen für dieses Phänomen symptomatisch zu sein; es werden in der Regel mit den ³¹P-NMR-Messungen deutlich höhere Anteile bestimmt als mit XRD bzw. mit XRD teilweise sogar keine Anteile an kristallinen Bestandteilen der letztgenannten Phosphate. Dies zeigt eindrucksvoll, warum die ³¹P-NMR-Messungen für die Charakterisierung und letztlich Herstellung der erfindungsgemäßen Werkstoffe eine wesentliche Voraussetzung bilden. Die XRD-Messungen erfolgten mit einem PW 1710, Philipps, NL (CuK radiation).
Es können daher sowohl kristalline als auch röntgenamorphe Phasen innig vermischt vorliegen. Für die vorliegende Erfindung ist es ohne Belang, ob eine Phase neben der anderen vorliegt, oder ob eine Phase die andere umhüllt. Als "Hauptkristallphase" wird hier eine über Röntgendiffraktion ermittelte kristalline Phase bezeichnet, deren Mengenanteil wenigstens doppelt so groß ist, wie der einer Nebenphase, wobei Konzentrationen von 25% und darunter, vorzugsweise unter 15 Gew-%, als Nebenphasen bezeichnet werden.

Überraschenderweise wurde nun gefunden, dass eine hohe Löslichkeit durch die Hauptphasen Ca₁₀Na(PO₄)₇, Ca₁₀K(PO₄)_{7'} und dazwischen liegende K-Na-Verhältnisse entsprechend den o.g. Formeln (Orthophosphate) und durch die Nebenphasen Na₂CaP₂O₇, K₂CaP₂O₇, Ca₂P₂O₇ (Diphosphate) erzielt werden kann, d.h. das relativ geringe Gehalte an Alkalien den gleichen Effekt bewirken, wie er von Zusammensetzungen in der Literatur bekannt ist, die ausschließlich Ca₂KNa(PO₄)₂ enthalten. Auch bewirkt eine zunehmende Substitution des Kaliums durch Natrium keinen abrupten Phasenübergang, wie es erfolgt, wenn man ausgehend vom Ca₂KNa(PO₄)₂ zwangsläufig zum Ca₅Na₂(PO₄)₄ gelangt, sondern es wechseln lediglich die Phasen Ca₁₀Na(PO₄)₇ und Ca₁₀K(PO₄)₇ ihre mengenmäßigen Anteile bzw. die möglicherweise gebildeten Mischkristalle zwischen diesen Stoffen gemäß Ca₁₀KₓNa₁₋ₓ(PO₄)₇ mit x=0 bis 1 ohne abrupten Phasenübergang.
x ist vorzugsweise 0,1 - 1, insbesondere 0,2 - 1.

Ferner wurde überraschenderweise gefunden, dass sich die Hauptkristallphasen Ca₁₀Na(PO₄)₇ und Ca₁₀K(PO₄)₇ und dazwischen liegende K-Na-Verhältnisse durch ³¹P-NMR-Messungen eindeutig charakterisieren lassen, und es im Gegensatz zum bislang angewendeten röntgendiffraktographischen Verfahren [XRD-Files: PDF-2 (1996) 450339 und 450138 ; Zh.Neoorg.Khim., 33(1988)73, inklusive der darin gegebenen Präparationsvorschriften für Ca₁₀Na(PO₄)₇ und Ca₁₀K(PO₄)₇] auch keine Verwechslungen bzw. Identifikationsprobleme mit der beta-TCP-Phase auftreten können. Die Anwesenheit der erfindungsgemäßen Hauptkristallphasen erklärt auch, warum diese glasig-kristallinen Materialien eine im Vergleich zum alpha- und/oder beta-TCP erheblich höhere Löslichkeit besitzen.

Die ³¹P-NMR-Messungen, die mit einem supraleitenden Fourier NMR Spektrometer Avance DMX400 WB der Fa. Bruker BioSpin GmbH (Deutschland) durchgeführt wurden, zeigten auch, dass das Material vorteilhaft aus 65 bis 99,9 % Orthophosphat besteht, gebildet aus Calcium sowie ggf. Natrium, Kalium und Magnesium wobei dieser Orthophosphat-Anteil nachweislich durch ³¹P-NMR-Messungen (Q₀-Gruppen) erfolgt und sich auf kristallines und/oder röntgenamorphes Material in seiner Gesamtheit bezieht, 0,1 bis 35% Diphosphat, gebildet aus Calcium sowie ggf. Natrium, Kalium und Magnesium wobei dieser Diphosphat-Anteil nachweislich durch ³¹P-NMR-Messungen (Q₁-Gruppen) erfolgt und sich auf kristallines und/oder amorphes Material in seiner Gesamtheit bezieht, 0 bis 15% Kettenphosphat, bestehend aus Natrium und/oder Kalium, wobei dieser Kettenphosphat-Anteil nachweislich durch ³¹P-NMR-Messungen (Q₂-Gruppen) erfolgt und sich insbesondere auf röntgenamorphes und ggf. mikrokristallines Material in seiner Gesamtheit bezieht. Ferner kann in Abhängigkeit vom gewählten SiO₂-Zusatz 0 bis 10 % einer Silicatphase enthalten sein.

Es wurde ferner überraschenderweise gefunden, dass die Anwesenheit von Di- bzw. Kettenphosphaten, vorzugsweise jedoch Diphosphaten, den gewünschten Effekt in Bezug auf eine deutliche Verbesserung der Löslichkeit bewirkt, wie das Beispiele 4 im weiteren belegen wird. Im Gegensatz zu dem im Abschnitt Stand der Technik zitierten alpha-TCP mit Dicalciumphosphat-Außenschicht, sind im erfindungsgemäßen Material alle Diphosphate mit den anderen Phasenbestandteilen "innig vermischt" und nicht schichtförmig aufgebaut, was eine hohe Löslichkeit bis zum vollständigen Verschwinden bzw. zur rückstandslosen Biodegradation bewirkt.

Die Diphosphatanteile resultieren aus einem vergleichsweise hohen Phosphatanteil im Vergleich zu den übrigen Bestandteilen. Dies könnte ursächlich dafür verantwortlich sein, dass die erfindungsgemäßen Zusammensetzungen im Vergleich zu bekannten resorbierbaren Werkstoffen sehr leicht einschmelzen und eine dünnflüssige Schmelze ergeben.

Ferner wurde überraschenderweise gefunden, dass durch die Anwesenheit von Diphosphaten nach Lagerung in deionisiertem Wasser der Werkstoff (das röntgenamorphe bzw. kristalline Material) nach anfänglich stark alkalischer Reaktion seinen lonenaustrag in Richtung physiologische pH-Werte (7,4) stärker verändert im Vergleich zu Werkstoffen, die kein Diphosphat enthalten. Dieser pH-Wert-Shift macht den Werkstoff auch für die Biotechnologie respektive Tissue Engineering interessant.

Dieses Merkmal lässt sich dadurch verstärken, dass die Formkörper (kompakt oder offenporig) durch Kochen in deinonisiertem Wasser (37 - 90°C) oberflächlich und gegebenenfalls auch unter Druck bis 10 bar ausgelaugt, so dass der auf diese Weise behandelte Werkstoff bzw. Formkörper nach dieser Behandlung deutlich geringere pH-Werte aufweist. Als Ursache dafür könnte eine Verarmung der alkalischen Ionen im oberflächennahen Bereich in Betracht gezogen werden. Eine solche Ausführungsform der Erfindung ist bevorzugt.

Ein Merkmal des erfindungsgemäßen Materials ist es, dass in Abhängigkeit von der gewählten Zusammensetzung die Löslichkeit in relativ weiten Bereichen eingestellt werden kann und zwar von 60 bis 250 µg/mg Gesamtlöslichkeit bezogen auf das Ausgangsmaterial, wenn die Prüfung in 0,2M TRIS-HCl-Puffer-Lösung bei pH=7,4, T=37°C, an einer Kornfraktion von 315 - 400µm, 120h lang bei einem Verhältnis von 50mg Probeneinwaage zu 40ml Pufferlösung erfolgte.

Ein weiteres Merkmal des Material ist es, dass es sich unter jeweils gleichen Mahlbedingungen (Pulverizette 5 der Firma Frisch GmbH, ZrO₂-Mahlbecher) feiner aufmahlen lässt als Werkstoffe die ausschließlich Ca₂KNa(PO₄)₂ als Hauptkristallphase enthalten [beschrieben in: Biomaterials 16 (1995)1241-1248]. Diese Eigenschaft, die möglicherweise durch einen hohen röntgenamorphen Anteil verursacht wird, ist insbesondere dann von Bedeutung, wenn aus dem Material spongiosa-artige Formkörper hergestellt werden sollen.

Erfindungsgemäß wird der Werkstoff hergestellt, indem die für die Gemengebildung geeignete Substanzen 35 - 55 Gew-% CaO, 30 - 50 Gew-% P₂O₅, 1 - 12 Gew-% Na₂O, 0,5 - 15 Gew-% K₂O und 0 - 5 Gew-% MgO sowie ggf. bis zu 5 Gew-% SiO₂ kombiniert werden, wobei MgO oder SiO₂ oder ein Gemisch davon wenigstens 1 Gew-% betragen und das Gemenge in mehrstufigen Temperaturbehandlungsprogrammen (Haltestufen im Bereich von 200 bis 1500°C, und zwar 1 - 2 h bei 350 - 450, 750 - 850 und 950 - 1050°C, z.B. jeweils 1 h bei 400, 800 und 1000°C, oder z.B. 1h bei 800°C und 1 h bei 950°C) in einem geeigneten Tiegelmaterial, zum Beispiel bestehend aus einer Pt/Rh-Legierung, bei 1550 bis 1650 °C zum Schmelzen gebracht werden. Die Schmelze kann 20 bis 60 min bei dieser Temperatur gehalten werden. Dann wird die Schmelze vergossen, und die erstarrte Schmelze wird je nach Verwendungszweck an der Luft (spontane Abkühlung) oder im Kühlofen mit temperaturprogrammierter Abkühlung von z.B. 1 bis 20 Grad/min auf Raumtemperatur abgekühlt. Während des Abkühlens der Schmelzen erfolgt eine spontane Kristallisation. Als Gemengebestandteile können Oxide, Carbonate, Hydrogenphosphate und/oder Ortho-Phosphorsäure verwendet werden. Die ³¹P-NMR-Messungen zeigen dabei Unterschiede in den Spektren auf, die auf die verwendeten Rohstoffe Rückschlüsse zulassen bzw. respektive deren geringfügige Beimengungen an Eisen- bzw. Manganoxiden.
Bevorzugte Schmelztemperaturen liegen bei 1590 - 1650°C.

Nach der Abkühlung wird der Werkstoff beispielsweise aufgemahlen, mit üblichen Sinterhilfsmitteln versetzt und sodann zu Formkörpern isostatisch verpresst, um nach dem Sintern einen möglichst dicht gebrannten keramischen Scherben zu erhalten.

Der erfindungsgemäß hergestellte Werkstoff kann auch beispielsweise aufgemahlen, mit üblichen Sinterhilfsmitteln versetzt und zu Schlicker verarbeitet werden, dieser auf einen Polyurethanschwamm aufgebracht und in mehreren Sinterstufen so hoch gesintert werden, dass der Polyurethanschwamm und die Sinterhilfsmittel ausgebrannt werden und ein spongiosa-artiger Formkörper mit den kristallinen Hauptbestandteilen an Ca₁₀Na(PO₄)₇, Ca₁₀K(PO₄)₇, ggf. Mischkristalle zwischen diesen beiden Phasen, sowie Na₂CaP₂O₇, K₂CaP₂O₇, Ca₂P₂O₇ erhalten wird.

In einer besonders vorteilhaften Ausführungsform der Erfindung ist es auch möglich, einen Teil der eingesetzten Rohstoffe separat zu schmelzen, um daraus ein Glas herzustellen, das als Sinterhilfsmittel wirkt und für die spongiosa-artigen Formkörper mit besonderem Vorteil verwendet werden kann. Dieses ebenfalls aufgemahlene Glas kann dabei dem Schlicker zugesetzt werden, der aus dem erfindungsgemäß hergestellten Werkstoff besteht, der zuvor nach dem Schmelzen und Abkühlen vermahlen und dann verschlickert wurde. In Bezug auf den Feststoffgehalt im Schlicker kann das separat geschmolzene Glas bis zu 15 Masse-% zugesetzt werden, so dass jedoch die einzelnen Komponenten nicht die erfindungsgemäßen Zusammensetzungsvorgaben überschreitet. Ein derartiges Glas kann insbesondere auf der Basis von SiO₂, MgO und Na₂O gebildet werden.
Beim Sinterprozess wird bei dieser Ausführungsform eine sehr feste Struktur des Formkörpers erreicht, während beim gemeinsamen Schmelzen aller Komponenten und späteren Sintern Abbröckeln des Formkörpers auftreten kann. Das separat geschmolzene Glas hat beim Zusetzen zu dem gemahlenen Werkstoff eine Korngröße D₅₀ von 0,7 bis 7 µm, wobei der Werkstoff etwa die gleiche oder eine größere Korngröße hat.

Ein Glas als Sinterhilfsmittel für resorbierbare calciumphosphathaltige Werkstoffe mit Ausnahme von β-TCP hat z.B. eine chemische Zusammensetzung in Gew-% von:
SiO₂: 73 - 78, vorzugsweise 74 - 75
MgO: 8 - 11, vorzugsweise 8,5 - 10
Na₂O: 12 - 19, vorzugsweise 14,5 - 17
K₂O: 0 - 22, vorzugsweise 0 - 5
P₂O₅: 0 - 20, vorzugsweise 0 -10.

Eine weitere Verarbeitungsmöglichkeit besteht darin, den Werkstoff aufzumahlen, mit üblichen Sinterhilfsmitteln zu versetzen und den so erhaltenen Schlicker zu einer Folie zu verarbeiten, die nach dem Brennprozess eine offenporige Struktur vorweist.

Vorteilhaft kann der erfindungsgemäße Werkstoff auch im Verbund mit einer metallischen Implantatoberfläche vorliegen. Da der Ausdehnungskoeffizient im Bereich von 10 bis 17x10⁻⁶K⁻¹ liegt, gemessen mittels Dilatometer (Kieselglas-Schubstangen-Dilatometer, Fa. Netzsch, Deutschland), ist eine Anpassung an bekannte Metalle, wie z.B Chrom-Kobalt-Molybdän-Stähle mit ähnlichen Ausdehnungskoeffizienten, besonders vorteilhaft.

Die Verwendung des erfindungsgemäßen glasig-kristallinen Materials zur Herstellung von Granulaten, keramischen Formkörpern oder keramischen Folien ist ebenfalls Gegenstand der Erfindung.

Die Erfindung wird nachstehend durch Beispiele näher erläutert. Alle Prozentangaben sind auf das Gewicht bezogen, sofern nicht anderes angegeben ist.

In der dazugehörigen Zeichnung zeigen
Fig.1: ³¹P-MAS-NMR-Spektren der erfindungsgemäßen Materialien 50-25-25 und 30-50-20 mit der Zusammensetzung gemäß Beispiel 2 und den Phasen gemäß Beispiel 5 (MAS = Magic Angle Spinning);
Fig. 2: ³¹P-MAS-NMR-Spektrum von β-TCP.

### Beispiel 1 (Vergleichsbeispiel)

Es wurden folgende Materialien synthetisiert nach den Vorgaben in Gew-%:

| Code | CaO | MgO | P₂O₅ | Na₂O | K₂O | SiO₂ |
|---|---|---|---|---|---|---|
| 100-0-0 | 30,67 | 2,45 | 43,14 | 9,42 | 14,32 | 0,00 |
| 97,5-0-2,5 | 29,92 | 2,39 | 44,53 | 9,19 | 13,97 | 0,00 |
| 95-0-5 | 29,21 | 2,33 | 45,85 | 8,97 | 13,64 | 0,00 |

Die Herstellung erfolgt dabei nach folgendem Prozedere:

Die Gemenge wurden wie folgt eingewogen:

| Code | CaCO₃ in g | MgO In g | 85%ige-H₃PO₄ in ml | Na₂CO₃ in g | K₂CO₃ in g | SiO₂ in g |
|---|---|---|---|---|---|---|
| 100-0-0 | 54,74 | 2,45 | 41,48 | 16,11 | 21,01 | 0 |
| 97,5-0-2,5 | 53,40 | 2,39 | 42,82 | 15,72 | 20,50 | 0 |
| 95-0-5 | 52,13 | 2,33 | 44,09 | 15,34 | 20,01 | 0 |

Zunächst werden die Komponenten an Calcium, Magnesium, Natrium und Kalium, ggf. auch Silicium eingewogen. Nach dem Einwiegen wird das jeweilige Gemenge in einem Taumelmischer eine Stunde lang gemischt. Danach wird das Gemenge mit der 85%igen Ortho-Phosphorsäure versetzt, gut gemörsert und gerührt sowie eine Stunden lang bei 100°C getrocknet, erneut gemörsert und wiederum eine Stunde bei 100°C im Trockenschrank aufbewahrt. Sodann wurde das Gemenge erneut gemörsert und in einen Pt/Rh-Schale gefüllt und auf 400°C erhitzt, nach Erreichen dieser Temperatur eine Stunde lang bei dieser Temperatur gehalten, sodann auf 800°C hochgeheizt, nach Erreichen dieser Temperatur wiederum eine Stunde lang bei dieser Temperatur gehalten und sodann auf 1000°C erhitzt und nach Erreichen dieser Temperatur eine Stunde lang bei dieser Temperatur gehalten. Dieser Sinterkuchen wurde an Luft abgekühlt und erneut zum Zwecke der Homogenisierung gemörsert. Dieses vorbehandelte Gemenge wurde sodann in einen Platin-Tiegel gefüllt und im Schmelzofen auf 1600°C erhitzt. Nach Erreichen dieser Temperatur wurde die Schmelze eine halbe Stunde lang bei dieser Temperatur belassen. Die dünnflüssigen homogenen Schmelzen wurden dann auf eine Stahlplatte gegossen und mit einer weiteren Stahlplatte zu einer salzartig erstarrten Platte verpresst. Die dabei erfolgende Kristallisation verleiht den Schmelzkörpern eine opake, weiße Farbe.

Röntgenuntersuchungen dieser Proben zeigten, dass diese Proben als Hauptkristallphase Ca₂K₁₋ₓNa₁₊ₓ(PO₄)₂ enthielten, die nicht Gegenstand der vorliegenden Erfindung ist, sondern einer gleichzeitig eingereichten Patentanmeldung der Erfinder. Es kann jedoch auch an diesen Beispielen die positive Wirkung von Diphosphatanteilen (vergleiche Werte im Beispiel 5) gezeigt werden in Bezug auf die Löslichkeit (vergleiche Werte im Beispiel 4). Mit steigenden Diphosphatanteil ergab sich hier eindeutig eine Erhöhung der Löslichkeit.

### Beispiel 2

Nach dem gleichen Herstellungsprozedere, wie in Beispiel 1 beschrieben, d.h. Gemengeerzeugung mit Calciumcarbonat, Natriumcarbonat, Kaliumcarbonat und Ortho-Phosphorsäure wurden folgende Zusammensetzungen nach den folgenden Vorgaben in Gew-% hergestellt:

| Code | CaO | MgO | P₂O₅ | Na₂O | K₂O | SiO₂ |
|---|---|---|---|---|---|---|
| 50-25-25 | 39,86 | 1,25 | 46,82 | 4,79 | 7,28 | 0 |
| 60-20-20 | 37,99 | 1,49 | 46,08 | 5,73 | 8,71 | 0 |
| 40-30-30 | 41,74 | 1 | 47,58 | 3,84 | 5,84 | 0 |
| 80-10-10 | 34,31 | 1,97 | 44,6 | 7,59 | 11,53 | 0 |
| 60-30-10 | 39,05 | 1,48 | 45,13 | 5,69 | 8,65 | 0 |
| 50-40-10 | 41,43 | 1,23 | 45,39 | 4,74 | 7,21 | 0 |
| 50-32,5-17,5 | 40,65 | 1,24 | 46,1 | 4,77 | 7,24 | 0 |
| 40-50-10 | 43,8 | 0,99 | 45,65 | 3,79 | 5,77 | 0 |
| 40-40-20 | 42,78 | 0,99 | 46,61 | 3,82 | 5,8 | 0 |
| 30-50-20 | 45,16 | 0,75 | 46,88 | 2,86 | 4,35 | 0 |
| 20-50-30 | 46,55 | 0,5 | 48,11 | 1,92 | 2,92 | 0 |
| 30-0-70 | 40,1 | 0,73 | 52,04 | 2,83 | 4,3 | 0 |
| 50-0-50 | 37,4 | 1,23 | 49,5 | 4,71 | 7,16 | 0 |
| 70-0-30 | 34,71 | 1,72 | 46,96 | 6,59 | 10,02 | 0 |
| 50-40-10-Si | 41,4 | 1,3 | 44,4 | 10,2 | 1,7 | 1 |

Es ergaben sich für alle Zusammensetzungskompositionen dünnflüssige Schmelzen, die spontan beim Abkühlen kristallisierten. Die Kristallisationsprodukte wiesen eine weiße Farbe auf.

### Beispiel 3

Eine weitere Herstellungsmöglichkeit besteht u.a. darin, dass ein Teil oder wie im vorliegenden Beispiel die gesamte Phosphor- bzw. Phosphatmenge durch einen Calciumträger eingebracht werden kann. Es wurde folgende Zusammensetzung synthetisiert nach den Vorgaben in Gew-%:

| Code | CaO | MgO | P₂O₅ | Na₂O | K₂O | SiO₂ |
|---|---|---|---|---|---|---|
| 60-20-20 | 37,99 | 1,49 | 46,08 | 5,73 | 8,71 | 0 |

Das Gemenge wurde wie folgt eingewogen:

| Code | CaCO₃ in g | MgO in g | 85%ige-H₃PO₄ in ml | Na₂CO₃ in g | K₂CO₃ in g | CaHPO₄ in g |
|---|---|---|---|---|---|---|
| 60-20-20 | 2,82 | 1,49 | 0,00 | 9,80 | 12,78 | 88,34 |

Das Gemenge wurde nach dieser Vorgabe eingewogen, eine Stunde lang im Taumelmischer gemischt und in einen Platin-Tiegel gefüllt und in einen auf 400°C vorgeheizten Ofen gestellt und 16 Stunden lang bei dieser Temperatur gehalten. Der Tiegel wurde entnommen und der Ofen nunmehr auf 600°C vorgeheizt, 4 Std. gehalten und der Ofen dann auf 950°C vorgeheizt. In dem auf 950°C vorgeheizten Ofen wurde der Tiegel 6 Stunden lang gehalten. Sodann wurde die Probe auf 1600°C hochgeheizt und eine halbe Stunde nach Erreichen dieser Temperatur bei dieser Temperatur gehalten. Die dünnflüssige homogene Schmelze wurde dann auf eine Stahlplatte gegossen und mit einer weiteren Stahlplatte zu einer salzartig erstarrten Platte verpresst. Die dabei erfolgende Kristallisation verleiht den Schmelzkörpern eine opake, weiße Farbe. In Abhängigkeit von der eingesetzten CaHPO₄-Komponente und deren Verunreinigung an Eisen und/oder Mangan, kann eine Verfärbung beobachtet werden.

Es ist auch möglich die Schmelze nach dem Schmelzvorgang (1600°C, 0,5h) direkt in einem Wasserbad abzuschrecken (Fritten), um im Falle einer weiteren Verarbeitung im Schlickerform das weitere Zerkleinern des Schmelzproduktes zu erleichtern.

### Beispiel 4

Zur Bestimmung der Löslichkeit wurde von den Proben nach Beispiel 1 und ausgewählten Proben nach Beispiel 2 (siehe folgende Tabelle) Granulate in der Kornfraktion 315µm bis 400µm hergestellt. Als Lösungsmittel wurde eine 0,2M TRIS-HCl-Puffer-Lösung mit pH=7,4 bei 37°C verwendet. Die geprüfte Menge betrug 50mg auf 40ml Lösungsmittel. Die Lagerung der Granulate erfolgte bei 37°C über eine Zeitdauer von 120h. Danach wurde die Gesamtlöslichkeit durch Bestimmung der Einzelionen (von Ca, Mg, P, Na, K) in der Lösung mit Hilfe einer ICP-Messung bestimmt zu:

| Code | Löslichkeit [µg/mg] |
|---|---|
| 50-25-25 | 187±10 |
| 60-20-20 | 164±14 |
| 40-30-30 | 160±15 |
| 80-10-10 | 108±8 |
| 60-30-10 | 123±11 |
| 50-40-10 | 123±7 |
| 50-32,5-17,5 | 127±22 |
| 40-50-10 | 105±16 |
| 40-40-20 | 152±4 |
| 30-50-20 | 121±14 |
| 20-50-30 | 78±4 |
| 100-0-0 | 95±8 |
| 97,5-0-2,5 | 134±16 |
| 95-0-5 | 221±22 |

Es ist überraschend, dass die Löslichkeitswerte der Zusammensetzungen nach Beispiel 2 im Vergleich zu denen nach Beispiel 1 eindrucksvoll hoch sind, obwohl die Summe der Alkali-Bestandteile, also bezogen auf Natrium- und Kaliumoxid, in den Zusammensetzungen nach Beispiel 2 deutlich geringer sind.

### Beispiel 5

Von den Proben nach Beispiel 1 und ausgewählten Proben nach Beispiel 2 wurden ³¹P-MAS-NMR-Spektren mit einer Wartezeit von 120s zwischen den Einzelpulsen aufgenommen. Die Probenrotationsgeschwindigkeit betrug 12,5 kHz.
Die quantitative Zusammensetzung der Proben in Bezug auf den Phosphatgehalt ist in folgender Tabelle angegeben:

| Code | Orthophosphat-Anteil [(PO₄)³⁻] in % | Diphosphat-Anteil [(P₂O₇)²⁻] in % | Kettenphosphat-Anteil [vorrangig (PO₃)¹⁻] in % |
|---|---|---|---|
| 50-40-10 | 92,5 | 7,5 | - |
| 50-32,5-17 | 93 | 7 | - |
| 50-25-25 | 86 | 14 | - |
| 40-50-10 | 91 | 9 | - |
| 40-40-20 | 84 | 16 | - |
| 40-30-30 | 82,5 | 13 | 4,5 |
| 30-50-20 | 88 | 9 | 3 |
| 20-50-30 | 73 | 27 | - |
| 60-20-20 | 92 | 8 | - |
| 100-0-0 | 99,5-96 | 0,5-4 | - |
| 97,5-0-2,5 | 88 | 12 | - |
| 95-0-5 | 79 | 21 | - |

Während von den angegebenen Proben jeweils nur eine Probe untersucht wurde, bezieht sich das Ergebnis mit der angegebenen Bandbreite für die Zusammensetzung 100-0-0 auf insgesamt drei Chargen, wobei eine Charge nach der unter Beispiel 3 genannten Herstellungsmethode synthetisiert wurde.

### Beispiel 6

In einer Planetenmühle im Zirkonoxidbecher (250ml) werden unter jeweils den gleichen Bedingungen (zwei Mal 20min lang) die geschmolzenen Produkte der Zusammensetzungen mit der Code-Bezeichnung 30-50-20, 40-30-30 und 60-20-20 sowie eine Zusammensetzung GB9/1 nach Biomaterials 16 (1995)1241-1248 aufgemahlen. Die Resultate sind der folgenden Tabelle zu entnehmen, wonach die erfindungsgemäßen Zusammensetzungen unter gleichen Mahlbedingungen zu kleineren Kornfraktionen führen:

| Code | D₅₀-Wert [in µm] |
|---|---|
| 30-50-20 | 4,21 |
| 40-30-30 | 3,98 |
| 60-20-20 | 3,67 |
| GB9/1 | 6,50 |

### Beispiel 7

Die aufgemahlene Probe 30-50-20 nach Beispiel 6 soll zu "scaffolds" verarbeitet werden. Von diesem gemahlenen Gut wurden 100g mit 45g eines Gemisches, bestehend aus 90% Polyethylenglykol und 10% eines handelsüblichen Netzmittels, unter Zugabe von 5ml Isopropylalkohol zu einem Schlicker verarbeitet. Dieser Schlicker wird auf PUR-Schwämme mit offener Porosität mit 31,49 - 7,87 Poren/cm (0 bis 20 Poren pro Zoll) durch mehrmaliges Eintauchen und Ausdrücken aufgebracht, im Trockenschrank über Nacht bei 120°C getrocknet und dann langsam mit 10°C pro Minute auf 1000°C erhitzt. Im Ergebnis ist ein spongiosa-artiges Material mit dem Ausgangsschwamm ähnlichem Aufbau vorhanden, und der PUR-Schwamm ist rückstandslos ausgebrannt.

### Beispiel 8

Die aufgemahlene Probe 60-20-20 nach Beispiel 6 soll zu "scaffolds" verarbeitet erden. Dies geschah nach der Methode von Beispiel 7. Das Ergebnis war insofern nicht vollständig zufriedenstellend, da die im Beispiel 7 erhaltene Probe augenscheinlich fester war. Um diesen Mangel auszugleichen, wurde als Sinterhilfsmittel 3-Masse-% eines zuvor hergestellten Glases mit der chemischen Zusammensetzung in Masse-% SiO₂: 74,97; MgO: 9,22 und Na₂O: 15,81 (eingeschmolzen als 27,04 Na₂CO₃) und einem D₅₀ von 6,56µm dem Mahlgut von 60-20-20 zugegeben. Von diesem Pulvergemisch wurden 100g mit 45g eines Gemisches, bestehend aus 90% Polyethylenglykol und 10% eines handelsüblichen Netzmittels, unter Zugabe von 5ml Isopropylalkohol zu einem Schlicker versetzt. Dieser Schlicker wird auf PUR-Schwämme mit offener Porosität mit 31,49 - 7,87 Poren/cm (80 bis 20 Poren pro Zoll) durch mehrmaliges Eintauchen und Ausdrücken aufgebracht, im Trockenschrank über Nacht bei 120°C getrocknet und dann langsam mit 10°C pro Minute auf 1000°C erhitzt. Im Ergebnis ist ein spongiosa-artiges Material mit dem Ausgangsschwamm ähnlichem Aufbau vorhanden, und der PUR-Schwamm ist rückstandslos ausgebrannt.

### Beispiel 9

Es wurden Proben nach Beispiel 2 hergestellt und davon ausgewählte Proben mit Hilfe der ³¹P-NMR untersucht. Die ³¹P-MAS-NMR-Spektren wurden mit einer Wartezeit von 120s zwischen den Einzelpulsen aufgenommen. Die Probenrotationsgeschwindigkeit betrug 12,5 kHz.

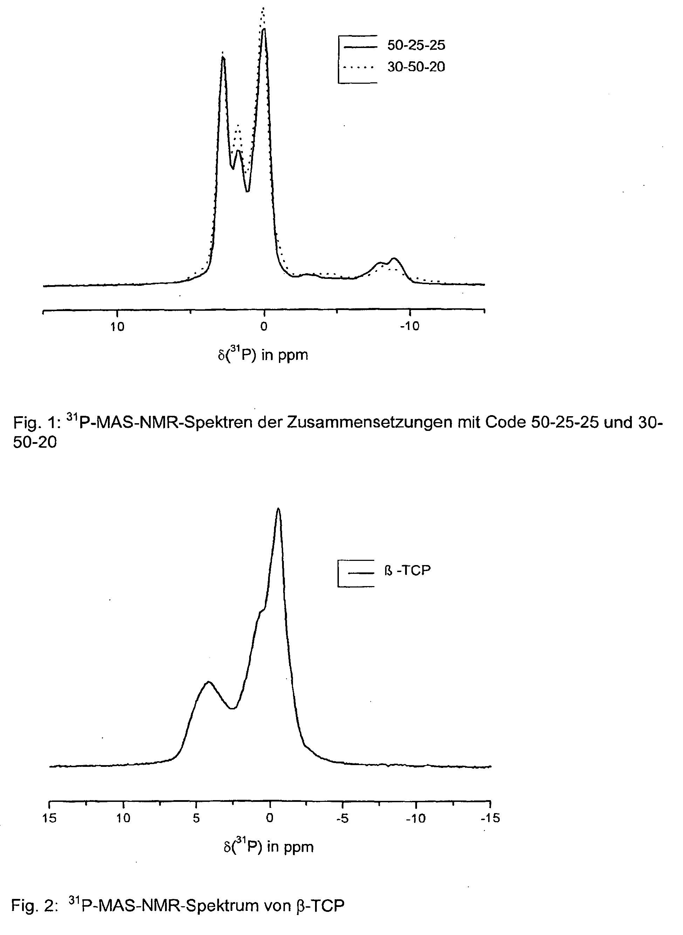

Im Ergebnis kann gezeigt werden, dass unter anderen Proben die Proben 50-25-25 sowie 30-50-20 (vergl. Fig. 1) im Gegensatz zu einer zertifizierten Referenzprobe bestehend aus β-TCP (Clarkson Chromatography Products, Inc., South Williamsport. PA, USA) (vergl. Fig. 2) die erfindungsgemäßen Proben überwiegend kein β-TCP enthalten, sondern den erfindungsgemäßen Kristallphasen Ca₁₀Na(PO₄)₇ bzw. Ca₁₀K(PO₄)₇ zuzuordnen sind. Möglicherweise existieren bislang noch nicht bekannte Mischkristallphasen zwischen den beiden Kristallphasen Ca₁₀Na(PO₄)₇ und Ca₁₀K(PO₄)₇, was eher wahrscheinlich als auszuschließen ist. Dies zu klären ist eine zukünftige wissenschaftliche Aufgabenstellung und hat auf die Herstellung und Verwendung des erfindungsgemäßen Werkstoffes keinen Einfluss.

In Fig 1 zeigt der linke Peak Q₀-Gruppen und der rechte höhere Peak Q₁-Gruppen.

Die erfindungsgemäßen Hauptphasen Ca₁₀Na(PO₄)₇ bzw. Ca₁₀K(PO₄)₇ bzw. dazwischen liegende Na-K-Verhältnisse konnten mit den NMR-Untersuchungen eindeutig als Q₀-Gruppen identifiziert werden. Es war auch eine eindeutige Zuordnung möglich, wenn als Hauptphasen Ca₁₀Na(PO₄)₇ bzw. Ca₁₀K(PO₄)₇ auftreten und β-TCP als Nebenphasenbestandteil.

### Beispiel 10

Material der Zusammensetzung mit der Code-Bezeichnung 30-50-20 wurde frisch aufgemahlen und 1g einer Kornfraktion von <45µm wurden in 100ml E-pur-Wasser gegeben und nach 1 min und nach 72h der pH-Wert bestimmt. Das Ergebnis nach einer Minute war 9,71 und nach 72 Stunden 8,3, d.h. deutlich in Richtung der physiologischen Bedingungen verschoben.

### Beispiel 11

Um diesen Effekt a priori zu verstärken, wurde folgender Versuch durchgeführt. Es wurde ein spongiosa-artiger Formkörper hergestellt nach Beispiel 7, d.h. also die Zusammensetzung der Code-Bezeichnung 30-50-20, und in dem vorliegenden Beispiel gesintert auf einen PUR-Ausgangsschwamm von 45ppi.
Dieser Formkörper von den äußeren Abmaßen ca. 12mmx10mmx6mm wurde in 100ml E-pur-Wasser gegeben und der pH-Wert nach 10min gemessen. Diese Messung ergab einen Wert von 8,25.

Sodann wurde der Formkörper bei 60°C und einem Druck von 3 bar ein E-pur-Wasser eine Stunde lang eluiert. Nach 5-fachen Spülen in jeweils 20ml frischem E-pur-Wasser wurde der Formkörper erneut in 100ml E-pur-Wasser gegeben und der pH-Wert nach 1h zu 7,84 und nach 4h zu 7,86 bestimmt.

Damit wird deutlich, dass eine derartige Vorbehandlung von spongiosa-artigen Formkörpern eine sinnvolle Verfahrensweise darstellt, da danach vorbehandelte Produkte eine geringere Basizität vorweisen, was sich sowohl bei der Implantation unter Bedingungen in vivo als auch beim Tissue Engineering unter Bedingungen ex vivo respektive in vitro als vorteilhaft erweisen kann.

### Beispiel 12

Im Hinblick auf eine Beschichtung von Werkstoffen mit diesen resorbierbaren erfindungsgemäßen Werkstoffen ist es von Bedeutung, dass der thermische Ausdehnungskoeffizient variiert werden kann, wenn man zum Beispiel bedenkt, dass Titanimplantate um 8•10⁻⁶ K⁻¹ und Co-Cr-Mo-Stähle um 14-16•10⁻⁶ K⁻¹ (abhängig von den Legierungsbestandteilen) aufweisen. Um ein für den jeweiligen Anwendungszweck optimalen Verbund zu erzeugen, spielt es eine Rolle, in welchem Temperaturbereich der Werkstoff auf das metallische Substrat aufgebracht wird, da man auf diese Weise auch gezielt das Substrat durch die Beschichtung unter Druckspannungen setzen kann, was im Allgemeinen als mechanisch stabilerer Verbund angesehen wird.
Die folgende Tabelle zeigt nun einen Ausschnitt aus der Variationsmöglichkeit:

| Probe | **AK** ₃₀₋₁₀₀ (10⁻⁶ K⁻¹) | **AK** ₃₀₋₂₀₀ (10⁻⁶ K⁻¹) | **AK** ₃₀₋₃₀₀ (10⁻⁶ K⁻¹) |
|---|---|---|---|
| 40-30-30 | 13,45 | 14,85 | 16,35 |
| 60-20-20 | 11,09 | 12,32 | 13,43 |
| 40-40-20 | 13,24 | 14,04 | 14,95 |
| 30-50-20 | 12,12 | 13,14 | 14,46 |
| 50-25-25 | 12,22 | 12,65 | 14,14 |

| | | | |
|---|---|---|---|
| AK₃₀₋₁₀₀ bedeutet dabei der Ausdehnungskoeffizient zwischen 30 und 100 °C, | | | |
| AK₃₀₋₂₀₀ ist der Ausdehnungskoeffizient zwischen 30 und 200 °C, und AK₃₀₋₃₀₀ ist der Ausdehnungskoeffizient zwischen 30 und 300 °C. | | | |

## Patentansprüche

1. Orthophosphathaltiger Knochenersatzwerkstoff , **dadurch gekennzeichnet, dass**
a) der Knochenersatzwerkstoff nach ³¹P-NMR-Messungen Q₀-Gruppen von Orthophosphat und Q₁-Gruppen von Diphosphat umfaßt, wobei die Orthophosphate respektive Q₀-Gruppen, bezogen auf den Gesamtphosphorgehalt des fertigen Werkstoffes, 65 bis 99,9 Gew-% betragen, und die Diphosphate respektive Q₁₋Gruppen, bezogen auf den Gesamtphosphorgehalt des fertigen Werkstoffes, 0,1 bis 35 Gew-% betragen, und
b) nach röntgendiffraktometrischen Messungen, bezogen auf das Gesamtgewicht des fertigen Werkstoffes, 35 bis 99,9 Gew-% einer Hauptkristallphase aus Ca₁₀Na(PO₄)₇, Ca₁₀K(PO₄)₇, Gemische davon oder Mischkristalle im Umfang von Ca₁₀KₓNa₁₋ₓ(PO₄)₇ mit x=0 bis 1 in dem Knochenersatzwerkstoff enthalten sind, und als Nebenkristallphase, bezogen auf das Gesamtgewicht des fertigen Werkstoffes, 0,1 bis 25 Gew-% eines Stoffes enthalten sind, ausgewählt aus der Gruppe, bestehend aus Na₂CaP₂O₇, K₂CaP₂O₇ , Ca₂P₂O₇ und Gemische davon, und
c) die röntgenamorphen Phasen neben der Hauptkristallphase insgesamt 0,1 bis 65 Gew-% betragen, bezogen auf das Gesamtgewicht des fertigen Werkstoffes, erhältlich durch Vermischen von Rohstoffen, enthaltend (in Gew-%) 30-50 CaO, 1-12 Na₂O, 0,5-15 K₂O, 0,1-5 MgO, 0-5 SiO₂ und Behandlung mit H₃PO₄ entsprechend einem Anteil von 35-55 P₂O₅, wobei SiO₂ oder MgO oder ein Gemisch davon wenigstens 1 Gew-% beträgt, das Gemenge homogenisert und getrocknet wird und einer stufenweisen Temperaturbehandlung von jeweils 1-2h bei 350-450 °C, 750-850 °C und 950-1050 °C unterworfen wird, und das Gemisch bei 1550 bis 1650 °C geschmolzen, bei der Schmelztemperatur 10 bis 60 Minuten gehalten und schließlich spontan oder mit geregelter Temperatur abgekühlt und gegebenenfalls gemahlen und zu Formkörpern gesintert wird.

2. Knochenersatzwerkstoff nach Anspruch 1, **dadurch gekennzeichnet, dass** zusätzlich ein oder mehrere Kettenphosphat(e) der Gruppe NaPO₃ , KPO₃ und Mischkristalle davon enthalten ist/sind, wobei die Kettenphosphatte nach ³¹P-NMR-Messungen als Q₂-Gruppen nachzuweisen sind, oder das Orthophosphat β-Tricalciumphosphat enthalten ist, das nach ³¹P-NMR-Messungen als Q₀-Gruppen nachzuweisen ist, oder Gemische davon.

3. Knochenersatzwerkstoff nach Anspruch 1, **dadurch gekennzeichnet, dass** der Anteil der Kettenphosphate 0,5 bis 10 Gew-% beträgt, vorzugsweise 0,5 bis 4 Gew-%.

4. Knochenersatzwerkstoff nach Anspruch 1, **dadurch gekennzeichnet, dass** in der Nebenkristallphase entsprechend dem SiO₂-Anteil eine Silicatphase enthalten ist.

5. Knochenersatzwerkstoff nach Anspruch 1, **dadurch gekennzeichnet, dass** in den kristallinen, amorphen oder in beiden Phasen Magnesium bis zu einem Anteil von 5 Gew-% , berechnet als MgO und bezogen auf das Gewicht des fertigen Werkstoffs enthalten ist.

6. Knochenersatzwerkstoff nach Anspruch 1, **dadurch gekennzeichnet, dass** der Anteil der Orthophosphate im Bereich von 40 bis 95 Gew-% liegt, vorzugsweise 50 bis 90 Gew-%.

7. Knochenersatzwerkstoff nach Anspruch 1, **dadurch gekennzeichnet, dass** der Anteil der Diphosphatphase im Bereich von 1 bis 22 Gew-% liegt, vorzugsweise im Bereich von 5 bis 8 Gew-%.

8. Knochenersatzwerkstoff nach Anspruch 1, **dadurch gekennzeichnet, dass** der Anteil der Nebenkristallphase 0,1 bis 25 Gew-% beträgt, vorzugsweise 1 bis 25 Gew-%.

9. Knochenersatzwerkstoff nach einem der Ansprüche 1 bis 8, **dadurch gekennzeichnet, dass** die Gesamtlöslichkeit, bezogen auf den Ausgangswerkstoff, im Bereich von 60 bis 250 µg/mg liegt, gemessen in 0,2M TRIS-HCl-Puffer-Lösung bei pH=7,4, T=37°C, an einer Kornfraktion von 315 - 400µm, 120h lang bei einem Verhältnis von 50mg Probeneinwaage zu 40ml Pufferlösung.

10. Knochenersatzwerkstoff nach einem der Ansprüche 1 bis 8, **dadurch gekennzeichnet, dass** der Ausdehnungskoeffizient im Bereich von 10 bis 17x10⁻⁶ K⁻¹ liegt, gemessen mittels Dilatometer.

11. Knochenersatzwerkstoff nach einem der Ansprüche 1 bis 8, **dadurch gekennzeichnet, dass** die Oberfläche nach Lagerung in deionisiertem Wasser bei Raumtemperatur über 72 Stunden oder Erhitzen auf 60 °C über 1 Stunde bei 1-3 bar und Spülen mit deionisiertem Wasser eine pH-Änderung im alkalischen Bereich um wenigstens 0,3 Einheiten, vorzugsweise wenigstens 1,4 Einheiten in Richtung zum Neutralpunkt zeigt.

12. Knochenersatzwerkstoff nach Anspruch 1, **dadurch gekennzeichnet, dass** der Werkstoff im Verbund mit einer metallischen Implantatoberfläche vorliegt.

13. Knochenersatzwerkstoff nach Anspruch 1, **dadurch gekennzeichnet, dass** er aus 44 bis 54 P₂O₅ , 34 bis 48 CaO, 1,5 bis 10,5 Na₂O, 1 bis 11 K₂O, 1,5 bis 3 MgO, 0,1 bis 4 SiO₂ besteht.

14. Knochenersatzwerkstoff nach Anspruch 1, **dadurch gekennzeichnet, dass** er in Form von Granulaten, keramischen Formkörpern oder keramischen Folien vorliegt.

15. Verfahren zur Herstellung eines orthophosphathaltigen Knochenersatzwerkstoffes nach Anspruch 1, **dadurch gekennzeichnet, dass** Rohstoffe, enthaltend (in Gew-%) 30-50 CaO, 1-12 Na₂O, 0,5-15 K₂O, 0,1-5 MgO, 0-5 SiO₂ vermischt und mit H₃PO₄ entsprechend einem Anteil von 35-55 P₂O₅ behandelt werden, wobei SiO₂ oder MgO oder ein Gemisch davon wenigstens 1 Gew-% beträgt, das Gemenge homogenisiert und getrocknet wird und einer stufenweisen Temperaturbehandlung von jeweils 1-2h bei 350-450 °C, 750-850 °C und 950-1050 °C unterworfen wird , und das Gemisch bei 1550 bis 1650 °C geschmolzen, bei der Schmelztemperatur 10 bis 60 Minuten gehalten und schließlich spontan oder mit geregelter Temperatur abgekühlt und gegebenenfalls gemahlen und zu Formkörpern gesintert wird.

16. Verfahren nach Anspruch 15, **dadurch gekennzeichnet, dass** die Rohstoffe in zwei Chargen aufgeteilt und separat geschmolzen werden, wobei die erste Charge aus einem geschmolzenen Glas aus 73-78 SiO₂, 8-11 MgO, 8,5-10 Na₂O, 12-19 K₂O und 0-22 P₂O₅ (in Gew-%) besteht, das nach dem Abkühlen aufgemahlen und in einem Anteil von 0,1-15 Gew-% der zweiten Charge, die nach dem Schmelzen das röntgenamorph-kristalline Material von Anspruch 15 darstellt, als Sinterhilfsmittel zugegeben wird und mit diesem zu Formkörpern bei 900-1200 °C gesintert wird.

17. Verfahren nach Anspruch 15, **dadurch gekennzeichnet, dass** das Gemisch bei 1590 bis 1650 °C geschmolzen wird.

## Claims

1. A bone replacement material with orthophosphate, **characterized in that**
a) according to ³¹P-NMR measurements, the bone replacement material comprises Q₀-groups of orthophosphate and Q₁-groups of diphosphate, the orthophosphates or Q₀-groups making up 65 to 99.9% by weight relative to the total phosphorus content of the finished material and the diphosphates or Q₁-groups making up 0.1 to 35% by weight relative to the total phosphorus content of the finished material, and
b) according to X-ray diffractometric measurements and relative to the total weight of the finished material, 35 to 99.9% by weight of a main crystal phase consisting of Ca₁₀Na(PO₄)₇, Ca₁₀K(PO₄)₇, mixtures thereof or mixed crystals according to the general formula Ca₁₀KₓNa₁₋ₓ(PO₄)₇, where x = 0 to 1, is contained in the bone replacement material and 0.1 to 25% by weight of a substance selected from the group consisting of Na₂CaP₂O₇, K₂CaP₂O₇, Ca₂P₂O₇ and mixtures thereof is contained as a secondary crystal phase, and
c) the X-ray amorphous phases contained besides the main crystal phase jointly make up 0.1 to 65% by weight relative to the total weight of the finished material, obtainable by mixing raw materials containing (in % by weight) 30-50 CaO, 1-12 Na₂O, 0.5-15 K₂O, 0-13 MgO and 0-10 SiO₂ and treating the aforesaid mixture with H₃PO₄ in an amount corresponding to 35-55 P₂O₅, SiO₂ or MgO or a mixture thereof making up at least 1% by weight, homogenizing and drying the mixture and subjecting it to a step-by-step thermal treatment lasting 1-2h at 350-450°C, 750-850°C and 950-1,050°C respectively, melting the mixture at between 1,550 and 1,650°C, holding it at the melting temperature for between 10 and 60 minutes and finally cooling the mixture in a spontaneous or temperature-controlled manner, grinding it, if necessary, and sintering it to obtain moulded bodies.

2. A bone replacement material according to Claim 1, wherein in addition one or more chain phosphates from the group consisting of NaPO₃, KPO₃ and mixed crystals thereof are contained, which chain phosphates are detectable as Q₂-groups according to ³¹P-NMR measurements, or the orthophosphate β-tricalcium phosphate, which can be detected as Q₀-groups according to ³¹P-NMR measurements, or mixtures thereof are contained.

3. A bone replacement material according to Claim 1, wherein the chain phosphates make up 0.5 to 10% by weight, preferably 0,5 to 4% by weight.

4. A bone replacement material according to Claim 1, wherein the secondary crystal phase contains a silicate phase corresponding to the SiO₂ content.

5. A bone replacement material according to Claim 1, wherein the crystalline, amorphous or both phases contain magnesium in an amount ranging up to 5% by weight, calculated as MgO and relative to the weight of the finished material.

6. A bone replacement material according to Claim 1, wherein the orthophosphates makes up 40 to 95% by weight, preferably 50 to 90% by weight.

7. A bone replacement material according to Claim 1, wherein the diphosphate phase makes up 1 to 22% by weight, preferably 5 to 8% by weight.

8. A bone replacement material according to Claim 1, wherein the secondary crystal phase makes up 0.1 to 25% by weight, preferably 1 to 25% by weight.

9. A bone replacement material according to any of Claims 1 through 8, wherein the total solubility ranges between 60 and 250µg/mg relative to the starting material if the test is carried out in 0.2M TRIS-HCl buffer solution at pH = 7.4, T = 37°C using a grain size fraction of 315-400µm, the duration of the test being 120h and the ratio of weighed-in sample to buffer solution being 50mg to 40ml.

10. A bone replacement material according to any of Claims 1 through 8, wherein the coefficient of expansion ranges between 10 and 17 x 10⁻⁶ K⁻¹, measured using a dilatometer.

11. A bone replacement material according to any of Claims 1 through 8, wherein the pH value of the surface changes by at least 0.3 units, towards the neutral point within the alkaline range if the material is stored in deionized water at room temperature for 72 hours or heated up to 60°C for 1 hour at a pressure of 1-3 bars and rinsed with deoinized water.

12. A bone replacement material according to Claim 1, wherein said material is provided in combination with a metallic implant surface.

13. A bone replacement material according to Claim 1, wherein said material consists of (in % by weight) 44 to 54 P₂O₅, 34 to 48 CaO, 1.5 to 10.5 Na₂O, 1 to 11 K₂O, 1.5 to 3 MgO, 0.1 to 4 SiO₂.

14. A bone replacement material according to Claim 1, wherein said material is provided in the form of granulated materials, ceramic bodies or ceramic sheets.

15. A method for manufacturing a bone replacement material comprising orthophosphate according to Claim 1, which comprises mixing raw materials containing (in % by weight) 25-30 CaO, 1-12 Na₂O, 0.5-15 K₂O, 0.1-5 MgO and 0-5 SiO₂ and treating the aforesaid mixture with H₃PO₄ in an amount corresponding to 35-55 P₂O₅, SiO₂ or MgO or a mixture thereof making up at least 1% by weight, homogenizing and drying the mixture and subjecting it to a step-by-step thermal treatment lasting 1-2h at 350-450°C, 750-850°C and 950-1,050°C respectively, melting the mixture at between 1,550 and 1,650°C, holding it at the melting temperature for between 10 and 60 minutes and finally cooling the mixture in a spontaneous or temperature-controlled manner, grinding it, if necessary, and sintering it to obtain moulded bodies.

16. A method according to Claim 15, wherein the raw materials are divided into two batches and melted separately, the first batch consisting of a melted glass comprising 73-78 SiO₂, 8-11 MgO, 8.5-10 Na₂O, 12-19 K₂O and 0-22 P₂O₅ (in % by weight), which glass is cooled, ground and added to the second batch, i.e. the X-ray amorphous-crystalline material obtained by the melting process of claim 15, in an amount ranging between 0.1 and 15% by weight as a sintering aid and is sintered jointly with said second batch at 900-1,200°C to obtain moulded bodies.

17. A method according to Claim 15, wherein the mixture is melted at between 1,590 and 1,650 °C.

## Revendications

1. Matériau de substitut osseux contenant de l'orthophosphate, **caractérisé en ce que**
a) le matériau de substitut osseux, d'après les mesures par résonance magnétique nucléaire (RMN) du phosphore 31, comprend des groupes Q₀ d'orthophosphate et des groupes Q₁ de diphosphate, sachant que les orthophosphates ou plutôt les groupes Q₀, en ce qui concerne la teneur totale en phosphore du matériau fini, s'élèvent entre 65 et 99,9 % en poids, et les diphosphates ou plutôt les groupes Q₁, en ce qui concerne la teneur totale en phosphore du matériau fini, s'élèvent entre 0,1 et 35 % en poids, et
b) d'après des mesures par diffractométrie des rayons X, en ce qui concerne le poids total du matériau fini, 35 à 99,9 % en poids d'une phase cristalline principale à base de Ca₁₀Na(PO₄)_{7'}, Ca₁₀K(PO₄)₇, de mélanges de ceux-ci ou de solutions solides dans une portée allant de Ca₁₀KₓNa₁₋ₓ(PO₄)₇ avec x=0 à 1, et comme phase cristalline secondaire, par rapport au poids total du matériau fini, 0,1 à 25 % en poids d'un matériau, choisi dans le groupe comprenant du Na₂CaP₂O₇, K₂CaP₂O₇, Ca₂P₂O₇ et des mélanges de ceux-ci, et
c) des phases amorphes aux rayons X, qui outre la phase cristalline principale s'élèvent au total à 0,1 à 65 % en poids, en ce qui concerne le poids total du matériau fini,
qui peut être obtenu par
mélange de matières premières, contenant (en % en poids) 30 à 50 CaO, 1 à 12 Na₂O, 0,5 à 15 K₂O, 1,5 à 5 MgO, 0 à 5 SiO₂ et un traitement avec du H₃PO₄ conformément à une teneur de 35 à 55 P₂O₅, sachant que SiO₂ ou MgO ou un mélange de ceux-ci s'élève à au moins 1 % en poids, le mélange est homogénéisé et est séché et est soumis à un traitement thermique progressif respectivement de 1 à 2 h à une température comprise entre 350 et 450°C, 750 et 850°C et 950 et 1050°C, et le mélange fond à une température comprise entre 1550 et 1650°C, est maintenu à la température de fusion pendant 10 à 60 minutes et est enfin refroidi spontanément ou à une température réglée et il est moulu le cas échéant et aggloméré par frittage en pièce moulée.

2. Matériau de substitut osseux selon la revendication 1, **caractérisé en ce qu'**une ou plusieurs chaîne(s) de phosphates du groupe NaPO₃, KPO₃ et des solutions solides de ceux-ci est/sont contenue(s) en plus, auquel cas les chaînes de phosphates doivent être détectées selon les mesures par RMN de phosphate 31 comme des groupes Q₂, ou de l'orthophosphate phosphate tricalcique β est contenu, qui doit être détecté selon les mesures par RMN de phosphate 31 comme des groupes Q₀, ou des mélanges de ceux-ci.

3. Matériau de substitut osseux selon la revendication 1, **caractérisé en ce que** la teneur en chaînes de phosphates s'élève entre 0,5 et 10 % en poids, de préférence 0,5 à 4 % en poids.

4. Matériau de substitut osseux selon la revendication 1, **caractérisé en ce que** conformément à la teneur en SiO₂ une phase silicate est contenue dans la phase cristalline secondaire.

5. Matériau de substitut osseux selon la revendication 1, **caractérisé en ce que** les phases soit amorphes soit cristallines ou les deux phases contiennent du magnésium dans une proportion allant jusqu'à 5 % en poids, calculée comme MgO et en fonction du poids du matériau fini.

6. Matériau de substitut osseux selon la revendication 1, **caractérisé en ce que** la teneur en orthophosphates se situe dans la plage comprise entre 40 et 95 % en poids, de préférence 50 à 90 % en poids.

7. Matériau de substitut osseux selon la revendication 1, **caractérisé en ce que** la proportion de la phase de diphosphate se situe dans la plage comprise entre 1 et 22 % en poids, de préférence dans la plage comprise entre 5 et 8 % en poids.

8. Matériau de substitut osseux selon la revendication 1, **caractérisé en ce que** la proportion de la phase cristalline secondaire s'élève entre 0,1 et 25 % en poids, de préférence de 1 à 25 % en poids.

9. Matériau de substitut osseux selon l'une quelconque des revendications 1 à 8, **caractérisé en ce que** solubilité totale relative à la matière première se situe dans une plage comprise entre 60 à 250 µg/mg, mesurée dans une solution tampon TRIS-HCI 0,2 M avec pH=7,4, T=37°C, pour une fraction granulométrique comprise entre 315 et 400 µm pendant 120 h avec un rapport de 50 mg de poids d'échantillon pour 40 ml de solution tampon.

10. Matériau de substitut osseux selon l'une quelconque des revendications 1 à 8, **caractérisé en ce que** le coefficient de dilatation se trouve dans une plage comprise entre 10 et 17x10⁻⁶ k⁻¹, mesuré au moyen du dilatomètre.

11. Matériau de substitut osseux selon l'une quelconque des revendications 1 à 8, **caractérisé en ce que** la surface présente, après suspension dans de l'eau déionisée à une température ambiante pendant 72 heures ou chauffée à 60°C pendant 1 heure avec une pression comprise entre 1 à 3 bar et après rinçage avec de l'eau déionisée, une modification de son pH dans la plage alcaline d'au moins 0,3 unité, de préférence d'au moins 1,4 unité en direction du point neutre.

12. Matériau de substitut osseux selon la revendication 1, **caractérisé en ce que** le matériau est disponible en liaison avec une surface d'implant métallique.

13. Matériau de substitut osseux selon la revendication 1, **caractérisé en ce qu'**il se compose de 44 à 54 P₂O₅, 34 à 48 CaO, 1,5 à 10,5 Na₂O, 1 à 11 K₂O, 1,5 à 3 MgO, 0,1 à 4 SiO₂.

14. Matériau de substitut osseux selon la revendication 1, **caractérisé en ce qu'**il est disponible sous la forme de granules, de pièces moulées céramiques ou de feuilles céramiques.

15. Procédé de fabrication d'un matériau de substitut osseux contenant de l'orthophosphate selon la revendication 1, **caractérisé en ce que** des matières premières, contenant (en % en poids) 30 à 50 CaO, 1 à 12 Na₂O, 0,5 à 15 K₂O, 1,5 à 5 MgO, 0 à 5 SiO₂ sont mélangées et traitées avec du H₃PO₄ conformément à une teneur de 35 à 55 P₂O₅, sachant que SiO₂ ou MgO ou un mélange de ceux-ci s'élève à au moins 1 % en poids, le mélange est homogénéisé et est séché et est soumis à un traitement thermique progressif respectivement de 1 à 2 h à une température comprise entre 350 et 450°C, 750 et 850°C et 950 et 1050°C, et le mélange fond à une température comprise entre 1550 et 1650°C, est maintenu à la température de fusion pendant 10 à 60 minutes et est enfin refroidi spontanément ou à une température réglée et il est moulu le cas échéant et aggloméré par frittage en pièce moulée.

16. Procédé selon la revendication 15, **caractérisé en ce que** les matières premières sont réparties en deux charges et fondues séparément, auquel cas la première charge se compose d'un verre fondu à base de 73 à 78 SiO₂, 8 à 11 MgO, 8,5 à 10 Na₂O, 12 à 19 K₂O et 0 à 22 P₂O₅ (en % en poids), qui est pulvérisé après le refroidissement et qui est ajoutée comme moyens de frittage dans une proportion de 0,1 à 15 % en poids de la deuxième charge, qui représente, après la fusion, le matériau cristallin amorphe aux rayons X de la revendication 15, et qui est frittée avec celui-ci en pièces moulées à une température comprise entre 900 et 1200°C.

17. Procédé selon la revendication 15, **caractérisé en ce que** le mélange fond à une température comprise entre 1590 et 1650°C.
